Europäisches Patentamt

European Patent Office

Office européen des brevets

(11) Publication number: **0 275 682 B1**

# (12) EUROPEAN PATENT SPECIFICATION

(45) Date of publication of patent specification: **21.11.91**   (51) Int. Cl.⁵: **A01G 7/00**

(21) Application number: **87311192.6**

(22) Date of filing: **18.12.87**

(54) Method of multiplicating bulbous plants.

(30) Priority: **18.12.86 JP 299964/86**

(43) Date of publication of application:
**27.07.88 Bulletin 88/30**

(45) Publication of the grant of the patent:
**21.11.91 Bulletin 91/47**

(84) Designated Contracting States:
**BE DE FR GB IT NL**

(56) References cited:
**EP-A- 0 150 528**
**US-A- 4 338 745**

(73) Proprietor: **MITSUI PETROCHEMICAL INDUS-
TRIES, LTD.**
**2-5, Kasumigaseki 3-chome Chiyoda-ku
Tokyo 100(JP)**

(72) Inventor: **Yamagata, Hikaru**
**2-1 Muronokicho 1-chome Iwakuni-shi
Yamaguchi 740(JP)**
Inventor: **Kawarabayashi, Waichirou**
**12-4 Shouzokucjo 5-chome Iwakuni-shi
Yamaguchi 740(JP)**
Inventor: **Takahashi, Shigeru**
**2-7 Misonio 1-chome Otakepshi
Hiroshima 739-06(JP)**

(74) Representative: **Davies, Jonathan Mark et al
Reddie & Grose 16 Theobalds Road
London WC1X 8PL(GB)**

## Description

The present invention relates to a method of multiplicating a great amount of the seedlings of bulbous plants by applying tissue culture to bulbous plants in a liquid culture medium.

There are various kinds of bulbous plants, for example, Lilium, in which L. longiflorum, L. speciosum, L. elegans, etc. have been favored for ornament as horticultural plants and, further, L. laneifolium L. auratum, etc. have been utilized as food lilies. The bulbous plants have hitherto been multiplicated by dividing bulbs, planting scales, utilization of bulbil, seeding, etc. However, these multiplicating methods require large areas and much labour, and as well there have been problems in view of the reduction of seedling growing rate or lowering in the flower quality of plants due to the spread of virus disease in recent years. In order to overcome these problems, several reports have been made of the study of multiplicating seedlings by tissue culturation of plants. For instance, Japanese Patent Laid-Open No. Sho 55-15734 discloses a method of multiplicating a great amount of Lilium plants by forming and multiplicating bulblets from the tissue pieces of lily plants using a solid culture medium, transplanting and then growing them in liquid culture medium. However, since a solid agar medium has been used at the former stage of the tissue culture in this method, it requires much labour for the culture. Also, effective space utilization for the culture vessel is difficult, leading to a lower culturing efficiency. US-A-4 338 745 discloses a method for propagating bulbous plants in which tissue pieces are cultured in a liquid culture medium.

In view of the above, the present applicant has already proposed a method of multiplicating lily seedlings by using a liquid culture medium from the start of the culture in which an oxygen-containing gas is permeated so as to keep the oxygen moving capacity coefficient (KLa) of the culture medium at a level within a predetermined range (Japanese Patent Application No. Sho 60-128348). This method has merit in that seedlings can be multiplicated in high efficiency as compared with the conventional method. Then, the present inventors have further improved this method and studied a method of multiplicating a great amount of seedlings, not restricted only to the lily plants but also to bulbous plants in general, capable of multiplicating the seedlings more efficiently and with more excellent shape for the bulblets and bulbous plants and further excellent in quality and stability.

As a result, we have accomplished the present invention based on the finding that the foregoing object can be attained by the following method. That is, the present invention provides a method of multiplicating bulbous plants in which seedlings of bulbous plants are multiplicated by applying tissue culture to tissue pieces or cultured cells of bulbous plants in a liquid culture medium, characterised in that the tissue culture is conducted while maintaining the osmotic pressure of the culture solution from 2 to 8 atm for substantially the entire period of the tissue culture.

These and other objects, as well as advantageous features of the present invention will become more apparent by reading the following descriptions in conjunction with accompanying drawings, wherein,

Figure 1 is a photograph of the bulbs of L. longiflorum obtained by the culture method according to the present invention;

Figure 2 is a photograph of the bulbs of L. longiflorum obtained in Comparative Example 1 in which the osmotic pressure of culture medium is kept at 0 atm during culture period;

Figure 3 is a graph showing the aging change of the osmotic pressure of the culture medium during culture in the examples of the present invention and in comparative examples.

The bulbous plants used in the tissue culture according to the present invention are those plants having scaly bulb as organs, eg: bulbs including flaky scale bulb and laminar scale bulb. The bulbous plants include specifically Lilium plants such as L. longiflorum, L. speciosum, L. elegans, L. laneifolium, L. auratum, etc.; Fritillaria plants such as F. camtschatcensis, F. imperialis, F. thunbergii, F. japonica, etc.; Hippeastrum hybridum (Hippeastrum plant); Narcissus plant such as N. pseudo-narissus; T. gesneriana having various varieties (Tulipa plants); H. orientalis (Hyacinthus plant); Allium plants such as A. schoneoprasum, A. ascalonicum, A. cepa, A. sativum, A. chinense, A. fitsulosum, A. giganteum, etc., the Lilium plants being preferred among them.

In the present invention, the tissue culture for a bulbous plant is conducted by using tissue pieces or cultured cells of the plant. The tissue pieces can include, specifically, those tissue pieces of bulbous plants prepared by finely slicing the tissues of shoot apex, stalks, leaves, flowers, seeds, bulbs, bulblets, scales, roots, etc. These tissue pieces are usually used after sterilizing with sodium hyperchlorite, ethanol or flame. However, such sterilizing procedures may not be necessary in the case of using aseptically cultured bulbous plants. Further, in the case of multiplicating the seedlings of bulbous plants not suffering from diseases and virus, the aforementioned tissue pieces obtained from the tissue near the apical point or the tissue pieces of the bulbous plants obtained from the tissues near the apical point can be used as the culture material. The cultured cells that can be used in the tissue culture for the bulbous plants according to the present invention are non-differentiated amorphous cells obtained from the tissue pieces by the tissue culture in the known method.

In the present invention, the tissue pieces or cultured cells of the bulbous plants described above are cultured under conditions in which the osmotic pressure of the culture solution is maintained within a range from 2 to 8 atm substantially over the entire period of the tissue culture.

In the tissue culture using a liquid culture medium employed so far, although the osmotic pressure in the fresh culture medium just after the start of the culture is usually as high as from 4 to 10 atm, the osmotic pressure is gradually lowered since the tissue pieces or cultured cells consume the nutrient ingredients in the culture medium along with the progress of the culture. In the tissue culture for the bulbous plants employed so far, no particular attention has been paid to the osmotic pressure during the culture period and the osmotic pressure at the later stage of the culture is rather lowered as compared with that at preceeding stages of the culture, that is, it is usually lowered to from 0 to 1 atm.

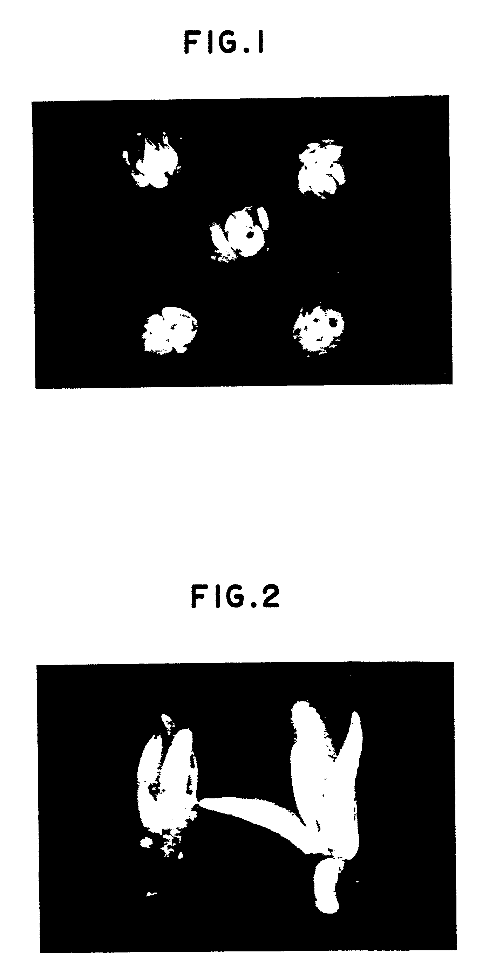

The present inventors have made a study of the culture conditions, particularly, the relationship between the osmotic pressure of the culture medium upon tissue culture of bulbous plants and the number of bulbs formed by the culture or the quality such as the shape of the bulblets, growing stability, etc. and, as a results, have found that the quality of the seedlings obtained in the culture is greatly influenced by the osmotic pressure of the culture solution during tissue culture. That is, in the case of the tissue culture for bulbous plants, particularly, in the latter stage of the culture, when culture is conducted by the usual way, in which the osmotic pressure of the culture medium is as low as less than 2 atm, the shape of the obtained bulbs or bulblets of bulb plants is not often the uniform spherical shape but takes the shape as shown, for example, in Figure 2, which is inferior both in shape and in view of the quality. Further, if the culture is conducted as previously under the osmotic pressure of the liquid culture medium, it has also been found that water content of bulblets or bulbs is usually as high as 80 - 95 %.

Further, if the bulbous plants are applied with tissue culture while increasing the osmotic pressure of the culture solution in excess of 8 atm, the number of the bulbs formed is reduced and the subsequent growing is poor to provide only low quality. While on the other hand, in a case where the culture is conducted while maintaining the osmotic pressure of the culture solution from 2 to 8 atm substantially over the entire culture period as in the present invention, it has been also found that the water content in the bulblets or bulbs obtained is as low as from 70 to 80 %.

It is considered that there is a relationship between the osmotic pressure of the culture solution used for the tissue culture of bulbous plants and the water content of the bulblets and the bulbs obtained by the culture. It is further considered that there is also a certain relationship between the water content and the quality such as the shape or the bulblets or bulbs, the growing stability, etc.

For the reasons described above, in the tissue culture for the bulbous plants according to the present invention, the culture is applied while maintaining the osmotic pressure of the culture solution at from 2 to 8 atm substantially over the entire period of the tissue culture. The substantially entire period for the tissue culture in this case will be described specifically later.

The liquid culture medium used in the present invention comprises those culture media containing inorganic ingredients as the nutrient ingredients, carbon sources and plant hormones as essential ingredients, as well as additives such as vitamins, amino acids, osmotic pressure controlling agents.

The inorganic ingredient for the culture medium can include, for example, those inorganic salts containing elements such as nitrogen, phosphorus, potassium, sodium, calcium, magnesium, sulfur, iron, manganese, zinc, boron, molybdenum, chlorine, iodine and cobalt. They can include, specifically, those compounds such as potassium nitrate, sodium nitrate, ammonium nitrate, ammonium chloride, potassium chloride, calcium chloride, potassium hydrogen phosphate, sodium dihydrogen phosphate, magnesium sulfate, magnesium chloride, sodium sulfate, ferrous sulfate, ferric sulfate, manganese sulfate, copper sulfate, sodium molybdate, molybdenum trioxide, potassium iodide, zinc sulfate, boric acid and cobalt chloride.

The carbon sources of the culture medium can include, for example, carbon hydrates such as sucrose, glucose, fructose, maltose and derivatives thereof.

Plant hormones for the culture medium can include, for example, auxines such as naphthalene acetic acid (NAA), indole acetic acid (IAA), p-chlorophenoxy acetic acid, 2,4-dichlorophenoxy acetic acid, 2,4-dichlorophenoxy acetic acid (2,4-D), indole butyric acid (IBA) and derivatives thereof, as well as cytokinin such as benzyl adenine (BA), kinetin and zeatin.

Vitamins for the culture medium can include, for example, biotine, thiamine (vitamin B1), pyridoxine (vitamin B6), pyridoxal, pyridoxamine, calcium pantotate, ascorbic acid (vitamin C), inositol, nicotinic acid, nicotinic amide and riboflavine (vitamin B2).

The amino acid for the culture medium can include, for example, glycine, alanine, glutamic acid, cystein, phenyl alanine and lysine.

The osmotic pressure controlling agent for the culture medium can include, for example, inorganic salts such as sodium chloride, potassium chloride, sodium sulfate, potassium sulfate, calcium chloride, calcium sulfate, sodium nitrate, potassium nitrate, calcium nitrate, ammonium nitrate and ammonium chloride, organic acid salts such as sodium acetate, potassium acetate, sodium propionate, potassium propionate, sodium citrate, potassium citrate, sodium maleate, potassium maleate, sodium malonate, potassium malonate, sodium tartarate, potassium tartarate, sodium succinate, potassium succinate, sodium fumarate, potassium fumarate, sodium citrate and potassium citrate. Carbon hydrate such as sucrose, glucose, fructose, maltose, mannose, galactose, xylose, ribose, arabinose, maldose, lactose, sorbitol, mannitol, galacturonic acid and gluconic acid, and water soluble higher alcohol such as ethylene glycol, propylene glycol, polyethylene glycol and dextrine. The osmotic pressure controlling agent may be the same as the inorganic ingredients and the carbon hydrates described above. Further, a plurality of them may be used in combination. The ratio of using the osmotic pressure controlling agent is usually within the range from 0 to 100 g/l for maintaining the osmotic pressure of the culture medium within the range from 2 to 8 atm.

The culture medium used usually contains from about 0.1 uM to about 100 uM of inorganic ingredients, from about 1 g/l to about 150 g/l of carbon source, from about 0.01 uM to about 1000 uM of plant hormones, from about 0.1 mg/l to about 150 mg/l of vitamins, from 0 to about 1000 mg/l of amino acid and the osmotic pressure controlling agent within the range as described above.

The culture medium used for the tissue culture according to the present invention can include specifically those known culture media used for the tissue culture, for example, Murashige & Skoog ('62) culture medium, Linsmaier & Skoog culture medium (RM-1965), White culture medium ('63), Gamborg B-5 culture medium, Mitsui M-9 culture medium, Nitch & Nitch culture medium, etc., being, preferably, incorporated as required with carbon sources and plant hormones as described above and, further, vitamins and amino acids as described above. The composition of the known culture media mentioned above are described, for example, in "New Plant Tissue Culture" p386 - p391 written by Takeuchi, Nakajima, Furuya, and published from Asakura Shoten in 1979. Preferably, the pH value for the culture medium is from 4.0 to 8.0. Although light is not always necessary during culture, better results may be obtained when culture is conducted under illumination of from 100 to 10000 lux. The temperature for the culture is preferably from 15 to 35 °C.

Description will now be made specifically of the method of conducting culture while maintaining the osmotic pressure within a range from 2 to 8 atm for the culture solution substantially over the former stage of the tissue culture period in the culture solution comprising the liquid culture medium as described above.

According to the present invention, a liquid culture medium is prepared in which the nutrient ingredients and, optionally, the osmotic pressure controlling agent as described above are contained at the concentration within the range described above and the osmotic pressure within the range from 2 to 8 atm in the liquid culture medium. The osmotic pressure is preferably controlled by adjusting the concentration of osmotic pressure controlling agents, such as the carbon hydrates such as sucrose, galactose, fructose, maltose, sorbitol, and mannitol. When the culture is carried out using the culture solution comprising a fresh liquid culture medium prepared by this method, since the osmotic pressure of the culture medium is lowered along with the consumption of the nutrient ingredients, the aging change of the osmotic pressure relative to the culture time is monitored, and the osmotic pressure of the culture solution is kept within the range as described above substantially over the entire period of the culture by adding appropriate amounts of nutrient ingredients and/or osmotic pressure controlling agent within the range of the concentration as described above. In this case, "substantially over the entire period of the culture" in the present invention means the following : osmotic pressure of the culture medium outside the range from 2 to 8 atm may be allowed if it occurs within such a period of time from the start of the culture, or just before the end of the culture, or in the intermediate period of the culture, so as to give no undesired effect on the tissue culture of bulbous plants - that is, over a period of time in such a range as not to impare the effect of the present invention; ie so that the shape of the bulblets and bulbs obtained by the culture is excellent, and a plurality of seedlings with stable growth and high quality can be obtained.

The time of period over which the effect of the present invention is not impaired by an osmotic pressure of the culture medium outside the range from 2 to 8 atm is usually about one-half day, although it may vary depending on the kind of plants.

Referring to the nutrient ingredients added to the culture medium in the present invention, the nutrient ingredients which are consumed during the period of the of the tissue culture are supplemented or replaced by addition of nutrient ingredients and/or osmotic pressure controlling agent in an amount sufficient to keep the osmotic pressure of the culture solution within the range as described above.

As the method of culturation while maintaining the osmotic pressure of the culture solution within the range from 2 to 8 atm substantially over the entire period of the tissue culture according to the present invention, it is possible to employ a culturing method while renewing the culture solution described below, beside the method as described above. That is, in the same way as the culturing method while renewing the culture solution as shown in Japanese Patent Application No. Sho 61-16838 filed by the present applicant, it is also possible to employ such a method of continuously or discontinuously applying a fresh liquid culture medium with the osmotic pressure from 2 to 8 atm to the culture solution in the culture tank substantially over the entire period of the tissue culture, while continuously or discontinuously extracting used culture solution from the culture tanks to renew the culture solution in the culture tank thereby culturing the bulbous plants while maintaining the osmotic pressure of the culture solution within the range from 2 to 8 atm over substantially the entire period of the tissue culture.

According to the present invention, the bulblets and the bulbs of bulbous plants obtained by the method as described above can be further separated into a plurality of scales or slices and it is possible to multiplicate a great amount of bulblets and the bulbs of the plants of excellent quality by the method according to the present invention.

In the method of applying tissue culture for bulbous plants by maintaining the osmotic pressure of the liquid culture medium within a range from 2 to 8 atm substantially over the entire period of the culture according to the present invention, it is possible to obtain a plurality of high quality seedlings excellent in the shape of the bublets and bulbs and in growth stability. By the method bulbous plants can be multiplicated more efficiently as compared with the conventional method.

EXAMPLES

The method of the present invention will be described more specifically referring to the Examples.

Example 1

Scales of bulbs of L. longiflorum were sterilized with 70 % ethanol and an aqueous solution of sodium hyperchlorite (effective chlorine concentration of 1 %) and then washed with aseptic water. Ab aseptic Linsmaier & Skoog culture solution at pH 6.0 containing 40 g/l of sucrose, 0.02 mg/l of naphthalene acetic acid and 80 g/l of sorbitol as the osmotic pressure controlling agent (the composition shown in Table 1) was diluted with two volumes of aseptic water to prepare twice-volume diluted solution. When the osmotic pressure of the culture solution was measured by the cryoscopic method, it was 7.5 atm. 50 scales and 250 ml of the culture solution as described above were placed in a culture tank with gas supply tube (500 ml volume) attached with glass filters and cultured in a dark place for 50 days at 25°C under aeration by passing aseptic air at a rate of 3 ml/min after passing through a sterilized filter.

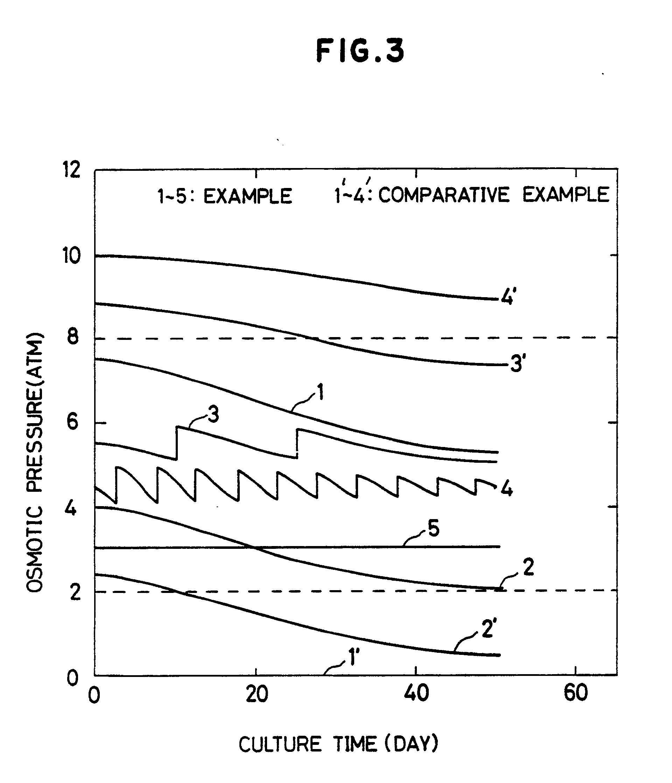

117 small bulblets of excellent shape as in Figure 1 were obtained. During the culture period, the osmotic pressure of the culture solution gradually dropped from 7.5 atm to 5.3 atm as shown in Figure 3. When 50 bulbs were dried at 40°C to examine the water content, it was 77 %. Remaining bulbs can be grown to flowering by the usual cultivation and were excellent in growing stability.

EP 0 275 682 B1

## Table 1  (Culture medium composition)

| Ingredient | Concentration (㎎／ℓ) | Ingredient | Concentration (㎎／ℓ) |
|---|---|---|---|
| KNO₃ | 1900 | | |
| NH₄NO₃ | 1650 | m-inositol | 100 |
| CaCl₂ · 2H₂O | 440 | glycine | 2 |
| MgSO₄ · 7H₂O | 370 | nicotinic acid | 0.5 |
| KH₂PO₄ | 170 | pyridoxine · HCl | 0.5 |
| Na₂EDTA | 37.3 | thiamine · HCl | 0.1- |
| FeSO₄ · 7H₂O | 27.8 | | |
| MnSO₄ · 4H₂O | 22.3 | | |
| ZnSO₄ · 7H₂O | 8.6 | | |
| H₃BO₃ | 6.2 | | |
| KI | 0.83 | | |
| Na₂MoO₄ · 2H₂O | 0.25 | | |
| CoCl₂ · 6H₂O | 0.025 | | |
| CuSO₄ · 5H₂O | 0.025 | | |

Example 2

The same procedures as in Example 1 were carried out except for adding sorbitol at 25 g/l. As a result, 120 bulblets of excellent shape were differentiated during culture for 50 days. During the culture period, the osmotic pressure of the culture solution gradually lowered from 4.0 atm to 2.1 atm as shown in Figure 3.

Example 3

The same procedures as in Example 1 were carried out except for adding 50 g/l of sorbitol and adding further sorbitol in an appropriate amount when the osmotic pressure of the culture solution during culture period becomes lower, thereby maintaining the osmotic pressure of the culture solution to 5.0 - 6.0 atm as shown in Figure 3. As a result, 120 bulblets showing stable growth and excellent shape were differentiated in 50 days culture.

Example 4

The same procedures as those in Example 1 were carried out except for adding 33 g/l of sorbitol, using 50 ml of the culture solution and supplying sorbitol and fresh culture solution appropriately so that the osmotic pressure of the culture solution was from 4.0 to 5.0 atm over the culture period as shown in Figure 3, while extracting used culture solution from the culture tanks. As the result, 119 bulblets of excellent shape were differentiated in 50 days culture.

6

Example 5

The same procedures as in Example 1 were carried out except for adding 8 g/l of sorbitol, using 50 ml of the culture solution and supplying sorbitol and fresh culture solution continuously so as to maintain the osmotic pressure of the culture solution at 3.0 atm during the culture, while continuously extracting used culture solution from the culture tanks as in Example 1. As a result, 123 bulblets of excellent shape were differentiated in 50 days culture.

Example 6

Scales of L. longiflorum bulbs were sterilized with 70 % ethanol and an aqueous solution of sodium hyperchlorite (effective chlorine concentration of 1 %) and then washed with aseptic water, and cut into about 2 mm widths. The same procedure as in Example 5 was conducted except for using 1 g of cut scales.120 to 150 bulblets of excellent shape were differentiated from 1 g of the sliced scales.

Example 7

Culture was conducted in the same manner as in Example 1 except for replacing the culture solution in Example 1 with a diluted solution prepared by diluting 1 x 1/3 times the Linsmaier & Skoog culture solution containing 50 g/l of sucrose, 0.01 mg/l of naphthalene acetic acid, 0.02 mg/l of benzyl adenine and 35 g/l of sorbitol as the osmotic pressure controlling agent (osmotic pressure : 7.0 atm). As a result, 123 bulblets of excellent shape were differentiated. The osmotic pressure of the culture solution gradually lowered from 7.0 atm to 3.7 atm during the culture.

Comparative Example 1

When carrying out the culture using sterilized water containing 0.01 mg/l of naphthalene acetic acid (osmotic pressure of 0 atm) instead of the culture medium as in Example 1, 107 bulblets were differentiated. Figure 2 shows a photograph of the obtained bulbs. The quality of the bulbs was inferior.

Comparative Example 2

When carrying out the culture in the same manner as in Example 1 using 50 aseptic scales obtained in the same manner as in Example 1 and 250 ml of aseptic Linsmaier & Skoog liquid culture medium at pH 6.0 containing 0.01 mg/l of naphthalene acetic acid and 30 g/l of sucrose (osmotic pressure of 0.1 atm), 113 bulblets were differentiated. The osmotic pressure of the culture medium after the culture was 1.3 atm. Most of the bulbs showed the shape as in photograph 2 and the quality of the bulbs was poor.

Comparative Example 3

The culture was carried out in the same manner as in Example 1 except for using sorbitol as the osmotic pressure controlling agent in an amount of 100 g/l in Example 1. As a result, 73 bulblets with unfavorable shape substantially the same as those shown in Figure 2 were differentiated. During the culture period, the osmotic pressure of the culture solution gradually lowered from 8.8 atm to 7.4 atm as shown in Figure 3. The number of the bulbs formed was reduced by 38 % as compared with Example 1.

Comparative Example 4

The same procedures as in Example 1 except for using the sorbitol as the osmotic pressure controlling agent in Example 1 in an amount of 120 g/l. As a result, 55 bulblets with undesirable shape substantially the same as that shown in Figure 2 were differentiated. During the culture period, osmotic pressure of the culture solution gradually lowered from 10.0 atm to 9.0 atm as shown in Figure 3. The number of the bulbs formed was reduced by 53 % as compared with Example 1.

**Claims**

1.  A method of multiplicating bulbous plants in which seedlings of the bulbous plants are multiplicated by applying tissue culture to tissue pieces or cultured cells of the bulbous plants using liquid culture medium, characterised in that the osmotic pressure of the culture solution is maintained at from 2 to 8 atm substantially over the entire period of the tissue culture.

2. A method of multiplicating bulbous plants as defined in claim 1, wherein the osmotic pressure is controlled by the addition of osmotic pressure controlling agent.

3. A method of multiplicating bulbous plants as defined in claim 1 or 2, wherein the tissue culture is carried out under the renewing condition for the culture solution.

4. A method of multiplicating bulbous plants as defined in claim 1 wherein nutrient ingredients consumed during the period of tissue culture are supplemented or replaced by addition of nutrient ingredients and/or osmotic pressure controlling agent.

**Revendications**

1. Procédé de multiplication de plantes à bulbes, dans lequel les plantes à bulbes sont multipliées en appliquant une culture de tissu sur des morceaux de tissu ou sur des cellules cultivées des plantes à bulbes, en employant un milieu de culture liquide, caractérisé en ce que la pression osmotique de la solution de culture est maintenue sous une pression sensiblement comprise entre 2 et 8 atm pendant toute la période de culture des tissus.

2. Procédé de multiplication des plantes à bulbes selon la revendication 1, dans lequel la pression osmotique est contrôlée en ajoutant un agent contrôleur de la pression osmotique.

3. Procédé de multiplication des plantes à bulbes selon l'une des revendications 1 ou 2, dans lequel la culture des tissus est effectuée dans des conditions de renouvellement de la solution de culture.

4. Procédé de multiplication de plantes à bulbes selon la revendication 1, dans lequel des produits nourrissants consommés pendant la période de culture des tissus sont complétés ou remplacés en ajoutant des produits nourrissants et/ou un agent contrôleur de la pression osmotique.

**Patentansprüche**

1. Verfahren zur Vermehrung von Zwiebelpflanzen, bei dem Sämlinge der Zwiebelpflanzen durch Anwendung einer Gewebekultur auf Gewebeteile oder kultivierte Zellen der Zwiebelpflanzen unter Anwendung eines flüssigen Nährmediums vermehrt werden, dadurch gekennzeichnet, daß der osmotische Druck der Nährlösung im wesentlichen über den gesamten Zeitraum der Gewebekultur bei 2 bis 8 atm gehalten wird.

2. Verfahren zur Vermehrung von Zwiebelpflanzen nach Anspruch 1, worin der osmotische Druck durch Zugabe eines Mittels zur Regelung des osmotischen Druckes geregelt wird.

3. Verfahren zur Vermehrung von Zwiebelpflanzen nach Anspruch 1 oder 2, worin die Gewebekultur unter Bedingungen zur Wiederherstellung der Nährlösung durchgeführt wird.

4. Verfahren zur Vermehrung von Zwiebelpflanzen nach Anspruch 1, worin die während des Zeitraumes der Gewebekultur verbrauchten Nährstoffbestandteile durch Zusatz von Nährstoffbestandteilen und/oder des Mittels zur Regelung des osmotischen Druckes ergänzt oder ersetzt werden.

# FIG.1

# FIG.2

# FIG.3